# EUROPEAN PATENT APPLICATION

(11) **EP 2 484 435 A1**
(43) Date of publication of application: **08.08.2012**
(21) Application number: 10820375.3
(22) Date of filing: 16.09.2010
(51) Int. Cl.: B01J 13/00, A61K 8/02, A61K 8/33, A61K 8/37, A61Q 1/14, A61Q 19/00, A61Q 19/10

(54) **GEL-LIKE COMPOSITION**

(30) Priority: 30.09.2009 JP 2009226037
(71) Applicant: Daicel Chemical Industries, Ltd., Kita-ku Osaka-shi Osaka 530-0001 (JP)
(72) Inventor: SAKANISHI, Yuichi, Ohtake-shi Hiroshima 739-0695 (JP)
(74) Representative: Grünecker, Kinkeldey, Stockmair & Schwanhäusser
(86) International application number: PCT/JP2010/066063
(87) International publication number: WO 2011/040256

(57) **Abstract**

Provided is a gel-like composition which has such satisfactory safety, storage stability, and versatility as to be widely used typically as cosmetics and pharmaceutical vehicles. The gel-like composition contains a polyglycerol monoalkyl ether represented by following Formula (1):

RO- (C₃H₆O₂) n-H (1)

wherein RO represents an alkoxy group having 12 to 22 carbon atoms, and "n" denotes a degree of glycerol polymerization and is 4 to 100
in a content of from 0.1 to 20 percent by weight; an aqueous component in a content of 0.65 to 1.5 times by weight relative to the content of the polyglycerol alkyl ether; and an oily component in a content of from 50 to 99.835 percent by weight.

## Description

### Technical Field

The present invention relates to a gel-like composition containing a polyglycerol monoalkyl ether with a superior level of safety. More specifically, the present invention relates to a gel-like composition useful typically as a cosmetic or a pharmaceutical vehicle (pharmaceutical base).

### Background Art

Gel-like compositions are heavily used typically in cosmetics, pharmaceutical vehicles, and foodstuffs. Customary gel-like compositions have widely employed, as surfactants, anionic surfactants such as alkyl sulfates, polyoxyethylene alkyl ether sulfate salts, polyoxyethylene alkyl ether phosphate salts, and α-olefin sulfonic acid salts. All these surfactants, however, more or less cause skin irritation, thus being problematic.

Non Patent Literature (NPL) 1 describes the use of a polyoxyethylene nonionic surfactant as the surfactant. However, the resulting gel-like composition may become inferior in gel formation ability because such a polyoxyethylene nonionic surfactant, as having an ethylene oxide chain as a hydrophilic group in the molecular chain, suffers from the decomposition of the ethylene oxide chain with time. In addition, the gel-like composition may suffer from dissolving out of formalin, a decomposed product of ethylene oxide, and may suffer from subsequent reduction in pH. The gel-like composition is therefore limited in use, thus being problematic.

Independently, the use of a polyglycerol fatty acid ester as the surfactant is investigated in a technique disclosed in NPL 2. However, it is difficult to industrially perform this technique, because the technique requires separation of hydrophilic groups through chromatography so as to obtain a higher molecular weight. Specifically, no gel-like composition has been found yet under present circumstances, which gel-like composition has such safety and versatility as to be widely used as cosmetics and pharmaceutical vehicles and can be easily industrially produced.

### Citation List

### Non Patent Literature

NPL 1: J.Colloid Inteerface Sci., 223,197(2000)
NPL 2: Journal of Japan Oil Chemists' Society, 49(6), 617(2000)

### Summary of Invention

### Technical Problem

Accordingly an object of the present invention is to provide a gel-like composition which has satisfactory levels of safety, storage stability, and versatility so as to be used in wide applications such as cosmetics and pharmaceutical vehicles and has excellent productivity.
Another object of the present invention is to provide a cosmetic containing the gel-like composition. Solution to Problem

After intensive investigations to achieve the objects, the present inventors have found that a specific polyglycerol monoalkyl ether is highly safe and, when used as a surfactant, gives a gel-like composition which can be easily prepared, acts mildly on the skin, and has excellent storage stability. They have also found that a gel-like composition, when containing the polyglycerol monoalkyl ether, an oily component, and an aqueous component in specific contents, can bear a cubic liquid crystalline phase having a three-dimensional higher order structure; and that this gel-like composition spreads well and gives good impression from use. The present invention has been made based on these findings.

Specifically, the present invention provides a gel-like composition which contains a polyglycerol monoalkyl ether represented by hollowing Formula (1):

RO- (C₃H₆O₂) n-H (1)

wherein RO represents an alkoxy group having 12 to 22 carbon atoms, and "n" denotes a degree of glycerol polymerization and is from 4 to 100,
in a content of from 0.1 to 20 percent by weight; an aqueous component in a content of from 0.65 to 1.5 times by weight relative to the content of the polyglycerol alkyl ether; and an oily component in a content of from 50 to 99.835 percent by weight.

In Formula (1), R is preferably lauryl group, myristyl group, palmityl group, or oleyl group.

The aqueous component is preferably water and/or a polyhydric alcohol.

The gel-like composition is preferably colorless and transparent.

The present invention further provides a cosmetic including the gel-like composition.

### Advantageous Effects of Invention

The gel-like composition according to the present invention uses, as a surfactant, the specific polyglycerol monoalkyl ether being safe and highly stable with time and can maintain the oily component as stably dispersed in a cubic liquid crystalline phase even when using substantially no other component. For this reason, the gel-like composition according to the present invention acts mildly on the skin, is extremely satisfactorily safe, and is easily prepared. The gel composition according to the present invention is particularly preferably used in cosmetics uses. Description of Embodiments

The gel-like composition according to the present invention contains a polyglycerol monoalkyl ether in a content of from 0.1 to 20 percent by weight; an aqueous component in a content of from 0.65 to 1.5 times by weight relative to the content of the polyglycerol alkyl ether; and an oily component in a content of from 50 to 99.835 percent by weight.

### [Polyglycerol Monoalkyl Ether]

The gel-like composition according to the present invention contains the polyglycerol monoalkyl ether in a content of from 0.1 to 20 percent by weight (preferably from 1 to 10 percent by weight). The polyglycerol alkyl ether, together with the aqueous component and the oily component, can form cubic liquid crystals which are surfactant aggregates in which micelles are packed in the form of cubic crystal, and this allows the formation of a very viscous, isotropic gel-like composition.

The polyglycerol monoalkyl ether for use in the present invention can be represented by following Formula (1) :

RO-(C₃H₆0₂)n-H (1)

wherein RO represents an alkoxy group having 12 to 22 carbon atoms, and "n" denotes a degree of glycerol polymerization and is from 4 to 100.

The moiety C₃H₆O₂ in the parentheses in Formula (1) has both structures represented by following Formulae (2) and (3) :

-CH₂-CHOH-CH₂O- (2)

-CH (CH₂OH) CH₂O- (3)

RO represents an alkoxy group having 12 to 22 carbon atoms. R represents an aliphatic hydrocarbon group having 12 to 22 carbon atoms, and examples thereof include linear alkyl groups such as n-dodecyl (n-lauryl), n-tridecyl, n-tetradecyl (n-myristyl), n-pentadecyl, n-hexadecyl, n-heptadecyl, n-octadecyl, n-nonadecyl, n-icosyl n-henicosyl, and n-docosyl groups; and linear alkenyl groups such as n-dodecenyl, n-tridecenyl, n-tetradecenyl, n-pentadecenyl, n-hexadecenyl, n-heptadecenyl, n-octadecenyl (n-oleyl), n-nonadecenyl, n-icosenyl, n-henicosenyl, and n-docosenyl groups.

Among them, linear alkyl groups or alkenyl groups each having 14 to 18 carbon atoms are preferred as R in the present invention, of which n-lauryl, n-myristyl, n-palmityl, or n-oleyl group is more preferred.

In Formula (1), "n" denotes a degree of glycerol polymerization and is from about 4 to about 100, preferably from 6 to 40, and particularly preferably from 8 to 20. If the repetition number "n" is less than the above range, micelles form hexagonal liquid crystals instead of the discontinuous cubic liquid crystals, and this may often impede the preparation of the gel. In contrast, if the repetition number "n" is more than the above range, the polyglycerol monoalkyl ether has excessively high water solubility to form a Zit10 (water-in-oil) emulsion, and this may often impede the preparation of the gel.

Exemplary polyglycerol monoalkyl ethers for use in the present invention include decaglycerol monomyristyl ether, decaglycerol monolauryl ether, decaglycerol monopalmityl ether, and decaglycerol monooleyl ether. Each of these may be used alone or in combination.

A process for producing the polyglycerol monoalkyl ether is not limited, and exemplary processes include (1) a process of adding glycidol to an aliphatic alcohol in the presence of a basic catalyst so that the ratio (by mole) of the aliphatic alcohol to glycidol be a specific value, and allowing them to react with each other; (2) a process of allowing a polyglycerol to react with an a-olefin epoxide; and (3) a process of ring-opening an alkyl glycidyl ether using a polyglycerol in the presence of an acid catalyst or an alkali catalyst. Among these processes, the process (1) is preferred in the present invention, from the viewpoint of the presence or absence of the formation of impurities.

### [Aqueous Component]

As used herein the term "aqueous component" refers to water, or a component that forms, when mixed with water, a uniform solution at any ratio. The aqueous component for use in the present invention is not limited and can suitably chose and employ an aqueous component of such quality as to correspond to the intended use such as cosmetics, pharmaceuticals, and foodstuffs. Examples thereof include water and polyhydric alcohols.

The water may be whichever of hard water and soft water and includes, for example, industrial water, tap (city) water, ion-exchanged water, distilled water, and purified water.

Exemplary polyhydric alcohols include glycerol, diglycerol, ethylene glycol, diethylene glycol, propylene glycol, polyethylene glycol, 1,3-butylene glycol, isoprene glycol, sorbitol, sorbitan, maltitol, trehalose, xylitol, glucose, erythritol, pentaerythritol, neopentyl glycol, sucrose, mannitol, gluconic acid, dipropylene glycol, hexylene glycol, and polyphenols. Each of these may be used alone or in combination.

Among them, water is preferably used as the aqueous component herein, and water may be used in combination with a polyhydric alcohol. The amount of the aqueous component to be incorporated may be suitably regulated depending on the intended use and is from about 0.65 to about 1.5 times by weight and preferably from 0.9 to 1.1 times by weight, relative to the content of the polyglycerol alkyl ether.

### [Oily Component]

The oily component for use in the present invention may be any one being in a liquid state at room temperature (20°C±15°C) and may be whichever of a natural oil/fat and a synthetic oil/fat. Exemplary oily components include saturated or unsaturated higher aliphatic hydrocarbons such as heptane, octane, decane, hexadecane, liquid paraffin, squalene, squalane, and pristane; animal and vegetable oils and fats such as avocado oil, camellia oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cottonseed oil, perilla oil, soybean oil, peanut oil, tea oil, kaya oil, rice bran oil, Chinese wood oil, Japanese wood oil (Japanese tung oil), jojoba oil, germ oil, and tall oil; higher alcohols such as octyldodecanol, oleyl alcohol, and myristyl alcohol; higher fatty acids such as oleic acid, myristic acid, and isostearic acid; silicone oils such as methylpolysiloxane, methylphenylpolysiloxanes, methylhydrogenpolysiloxanes, decamethylpolysiloxanes, and decamethylcyclopentasiloxane; and esters such as cetyl octanoate, isopropyl myristate, isopropyl palmitate, hexyl laurate, oleyl oleate, decyl oleate, octyldodecyl myristate, hexyldecyl dimethyloctanoate, diethyl phthalate, dibutyl phthalate, glycerol trioctanoate, glycerol triisopalmitate, triglyceride 2-ethylhexanoate, and triglyceride caprate. Oils and fats for use in the present invention may also be those obtained by subjecting the above-mentioned oils and fats to a treatment such as hydrogenation or separation. Each of these may be used alone or in combination.

The amount of the oily component to be incorporated is generally from 50 to 99.835 percent by weight, and preferably from 60 to 95 percent by weight, in terms of percent by weight relative to the weight of the resulting (final) composition.

The gel-like composition according to the present invention may further contain one or more other components as appropriate within such a range as to attain the objects of the present invention, in addition to the polyglycerol monoalkyl ether, the aqueous component, and the oily component. Exemplary other components include drugs or agents, antiseptics, colorants, flavoring agents, humectants, ultraviolet absorbers, essential oils, waxes, fatty acids and alcohol esters of them, vitamins, antioxidants, saturated or unsaturated higher alcohols, hydrocarbons, preservatives, and antimicrobial agents, which are generally used typically in cosmetics and pharmaceuticals.

The gel-like composition according to the present invention may be prepared, for example, through the following steps.
(1) Initially, the polyglycerol monoalkyl ether and the aqueous component are mixed with each other in a suitable ratio to give a micellar solution for the formation of cubic liquid crystals. As used herein the term "micelle" refers to an assembly or aggregate in which the surfactant molecules aggregate so that hydrophilic groups face water, and hydrophobic groups face in.
(2) The resulting micellar solution is combined with the oily component, followed by stirring. This can form a gel-like composition in which the oily component is contained in a cubic liquid crystalline phase. The stirring may be performed by shaking or by intense mechanical agitation. Among them, intense mechanical agitation is preferred in the present invention.

Exemplary self-assembled articles that are capable of forming a gel include cubic liquid crystals, lamellar liquid crystals, and hexagonal liquid crystals. In the gel-like composition according to the present invention, the polyglycerol monoalkyl ether forms optically isotropic cubic liquid crystals, and thereby the gel-like composition can contain the oily component in a large amount to form a highly viscous gel. In addition, the gel-like composition can contain the oily component as dispersed stably and can maintain a stable gel state without phase separation, because the oily component is contained in the cubic liquid crystalline phase.

Cubic liquid crystals are known to be categorized by structure into four types, i.e., a discontinuous micellar cubic phase (I1) in which micelles are packed in the form of cubic crystal; a normal bicontinuous cubic phase (VI) having a structure where both water and surfactant aggregates are continuous, and the surfactant molecular layer has a somewhat positive curvature; a reverse bicontinuous cubic phase {it2} which has the same structure as with VI, except that the surfactant molecular layer has a somewhat negative curvature; and a discontinuous reverse micellar cubic phase (I2) in which spherical reversed micelles are packed in the form of cubic crystal. As used herein a "positive" curvature of a surfactant molecular layer means that the layer is convex toward water, and a "negative" curvature means that the layer is concave toward water. The cubic liquid crystal in the present invention may be any of the four types of phases but is preferably of the discontinuous micellar cubic phase (I1).

The gel-like composition according to the present invention may be either colorless and transparent, or semitransparent, but is preferably colorless and transparent (clear) from the viewpoint of product image. As used herein the term "colorless and transparent" refers to that the article in question has a total light transmittance of 80% or more. The gel-like composition can have higher transparency by suitably regulating the compositional ratios of respective components.

The gel-like composition according to the present invention excels in safety and storage stability. In addition, it spreads well, acts mildly on the skin, and has excellent impression from use. For these reasons, the gel-like composition according to the present invention can be widely used, without limitation, in various products which are in a gel-form at room temperature (20°C±15°C), such as cosmetics, deodorants, bath additives, flavoring agents (aromatics), deodorizers, foodstuffs, and pharmaceuticals. The gel composition according to the present invention is advantageously usable in applications including cosmetics such as cosmetics for oil supplementation, moisturizing cosmetics, cosmetics for blood circulation promotion, detergents, bath cosmetics, cleansing products (make-up removers), massage agents, pack cosmetics, and hair cosmetics; and pharmaceutical vehicles such as vehicles for dermatological pharmaceuticals. Above all, the gel-like composition is suitable for cosmetics uses.

### EXAMPLES

The present invention will be illustrated in further detail with reference to several working examples below. It should be noted, however, that these examples are never construed to limit the scope of the present invention.

### EXAMPLES 1 TO 10 AND COMPARATIVE EXAMPLES 1 TO 8

A surfactant and an aqueous component were mixed with each other at room temperature (25°C) in proportions (percent by weight) given in Table 1 below, and the mixture was thereafter combined with an oily component added gradually at room temperature (25°C) with stirring using a homogenizer. Thus, a series of transparent gel-like compositions was obtained.

### <X-Ray Diffraction>

In the examples and comparative examples above, gel-like compositions in the midway of their production, namely, gel-like compositions to which the oily component had been added to an amount of about 10 percent by weight, were subjected to measurements of scattering angle using a conformal X-ray scattering system (trade name "SWXD", supplied by Rigaku Corporation), and data were evaluated according to the following methods.

### Evaluation Criteria:

Discontinuous micellar cubic phase (11) is formed: Good
Discontinuous micellar cubic phase (is) is not formed: Poor

### <Storage Stability>

Each of the gel-like compositions obtained in the examples and comparative examples was sealed in a glass tube having a diameter of 10 mm and a length of 8 cm, was stored while being immersed in an isothermal water bath set at 25°C, wether the gel-like composition in the glass tube was separated or not was visually observed, and the storage stability was evaluated according to the following criteria.

### Evaluation Criteria:

No reparation of the oily component is observed: Good Separation of the oily component is observed: Poor

**[Table 1]**

| TABLE 1 | | | | | | | | | | | | | | | | | | | |
|---|---|---|---|---|---|---|---|---|---|---|---|---|---|---|---|---|---|---|---|
| Components | | Examples | | | | | | | | | | Comparative Examples | | | | | | | |
| | | 1 | 2 | 3 | 4 | 5 | 6 | 7 | 8 | 9 | 10 | 1 | 2 | 3 | 4 | 5 | 6 | 7 | 8 |
| Oily component | Cetyl octanoate | 90 | | | 90 | 90 | 90 | 90 | 90 | 90 | 90 | 90 | 90 | 90 | 90 | 90 | 90 | 90 | 90 |
| | Liquid paraffin | | 90 | | | | | | | | | | | | | | | | |
| | Decamethylcyclopentasiloxane | | | 90 | | | | | | | | | | | | | | | |
| Surfactant | Decaglycerol monomyristyl ether | 5 | 5 | 5 | | | | 6 | 4 | | | | | | | 7 | 3 | | |
| | Decaglycerol monolauryl ether | | | | 5 | | | | | 6 | 4 | | | | | | | 7 | 3 |
| | Decaglycerol monopalmityl ether | | | | | 5 | | | | | | | | | | | | | |
| | Decaglycerol monooleyl ether | | | | | | 5 | | | | | | | | | | | | |
| | Decaglycerol monooctyl ether | | | | | | | | | | | 5 | | | | | | | |
| | Decaglycerol monodecyl ether | | | | | | | | | | | | 5 | | | | | | |
| | Decaglycero, monolaurate | | | | | | | | | | | | | 5 | | | | | |
| | Decaglycerol monomyristate | | | | | | | | | | | | | | 5 | | | | |
| Aqueous component | Purified water | 5 | 5 | 5 | 5 | 5 | 5 | 4 | 6 | 4 | 6 | 5 | 5 | 5 | 5 | 3 | 7 | 3 | 7 |
| Evaluation | X-ray diffraction | Good | Good | Good | Good | Good | Good | Good | Good | Good | Good | Good | Good | Good | Good | Good | Good | Good | Good |
| results | Storage stability | Good | Good | Good | Good | Good | Good | Good | Good | Good | Good | Poor | Poor | Poor | Poor | Poor | Poor | Poor | Poor |

### Industrial Applicability

The gel-like composition according to the present invention uses a specific polyglycerol monoalkyl ether as a surfactant and can thereby maintain an oily component as being stably dispersed in a cubic liquid crystalline phase. The gel-like composition therefore acts mildly on the skin, is extremely highly safe, and can be easily prepared. The gel composition according to the present invention is advantageously usable in applications including cosmetics such as cosmetics for oil supplementation, moisturizing cosmetics, cosmetics for blood circulation promotion, detergents, bath cosmetics, cleansing products (make-up removers), massage agents, pack cosmetics, and hair cosmetics; and pharmaceutical vehicles such as vehicles for dermatological pharmaceuticals.

## Claims

1. A gel-like composition comprising a polyglycerol monoalkyl ether represented by following Formula (1) :
RO-(C₃H₆0₂)n-H (1)
wherein RO represents an alkoxy group having 12 to 22 carbon atoms, and "n" denotes a degree of glycerol polymerization and is from 4 to 100,
in a content of from 0.1 to 20 percent by weight; an aqueous component in a content of from 0.65 to 1.5 times by weight relative to the content of the polyglycerol alkyl ether; and
an oily component in a content of from 50 to 99.835 percent by weight.

2. The gel-like composition according to claim 1, wherein R in Formula (1) is lauryl group, myristyl group, palmityl group, or oleyl group.

3. The gel-like composition according to claim 1 or 2,
wherein the aqueous component is water and/or a polyhydric alcohol.

4. The gel-like composition according to any one of claims 1 to 3, which is colorless and transparent.

5. A cosmetic comprising the gel-like composition of any one of claims 1 to 4.
